## Europäisches Patentamt
## European Patent Office
## Office européen des brevets

(19)

(11) Veröffentlichungsnummer: **0 117 496**
**B1**

(12) # EUROPÄISCHE PATENTSCHRIFT

(45) Veröffentlichungstag der Patentschrift:
**08.06.88**

(21) Anmeldenummer: **84101657.9**

(22) Anmeldetag: **17.02.84**

(51) Int. Cl.⁴: **B 01 J 23/62,** C 07 C 67/39,
C 07 C 69/54

(54) **Katalysator und seine Verwendung zur Herstellung von Methylmethacrylat.**

(30) Priorität: 26.02.83 DE 3306907

(43) Veröffentlichungstag der Anmeldung:
05.09.84 Patentblatt 84/36

(45) Bekanntmachung des Hinweises auf die Patenterteilung:
08.06.88 Patentblatt 88/23

(84) Benannte Vertragsstaaten:
BE DE FR GB IT NL

(56) Entgegenhaltungen:
EP-A-0 089 587
BE-A-699 551
US-A-3 917 676
US-A-4 130 597
US-A-4 249 019

(73) Patentinhaber: BASF Aktiengesellschaft, Carl-Bosch- Strasse 38, D-6700 Ludwigshafen (DE)

(72) Erfinder: Baer, Karl, Dr., Kastanienweg 1, D-6940 Weinheim (DE)
Erfinder: Bassler, Peter, Dr., Luisenstrasse 7, D-6945 Hirschberg (DE)
Erfinder: Duembgen, Gerd, Dr., Sudetenstrasse 4, D-6701 Dannstadt- Schauernheim (DE)
Erfinder: Fouquet, Gerd, Dr., Maxburgstrasse 2, D-6730 Neustadt (DE)
Erfinder: Krabetz, Richard, Dr., Unterer Waldweg 8, D-6719 Kirchheim (DE)
Erfinder: Merger, Franz, Dr., Max- Slevogt- Strasse 25, D-6710 Frankenthal (DE)
Erfinder: Nees, Friedbert, Dr., Fichtestrasse 4, D-7513 Stutensee (DE)

LIBER, STOCKHOLM 1988

**Beschreibung**

Die Herstellung von Carbonsäureestern aus Aldehyden und Alkoholen in Gegenwart von Sauerstoff unter dem Einfluß von Katalysatoren ist ein seit einiger Zeit von mehreren Seiten bearbeitetes Verfahren. Für dieses Verfahren sind verschiedenartige Katalysatoren vorgeschlagen worden, von denen besonders solche, die als aktiven Bestandteil Palladium enthalten, auf Interesse gestossen sind. Die bisher bekanntgewordenen Katalysatoren dieser Art haben aber noch nicht alle Wünsche voll befriedigen können, vor allem, soweit sie zur Herstellung von Estern α,β-olefinisch ungesättigter aliphatischer Carbonsäuren, wie insbesondere Methylmethacrylat (im folgenden auch "MMA" genannt), verwendet werden.

Aus der US-A-3 772 381 ist es bekannt, für die Umsetzung α, β-olefinisch ungesättigter aliphatischer Aldehyde mit niederen ein wertigen primären oder sekundären Alkanolen und molekularem Sauerstoff zu Estern der entsprechenden Carbonsäuren als Katalysator metallisches Palladium zu verwenden, das gegebenenfalls auf einem geeigneten Träger, wie besonders Aluminumoxid oder Siliciumdioxid, aufgebracht ist. Dieser Katalysator hat den Nachteil, daß er erhebliche Mengen an Nebenprodukten (Methylformiat 27 Gew.-% und Formaldehyd 18 Gew.-%, bezogen auf Methylmethacrylat) liefert. Außerdem werden nur geringe Umsätze oder nur geringe Selektivitäten erreicht, da man aus eingesetzten 3,58 mol Methacrolein/1 Katalysator pro h lediglich 0,11 mol Methylmethacrylat/1 Katalysator pro h erhält (vgl. dort Beispiel 4).

Die US-Patentschrift 3 639 449 beschreibt ein sehr ähnliches Verfahren zur Darstellung von Carbonsäureestern aus Aldehyden und/oder Alkanolen durch Reaktion mit Sauerstoff an Edelmetall-Katalysatoren (z. B. Palladium) bei 0 bis 300°C. Auch hier zeigt sich die mangelhafte Brauchbarkeit eines reinen Palladiumkatalysators für die Herstellung von Methylmethacrylat: Beispiel 16, das einzige, das die Darstellung von Methylmethacrylat aus Methanol und Methacrolein an einem Katalysator (2 % Pd auf Aktivkohle) erläutert, gibt einen Methacrolein-Umsatz von 17,3 % an mit einer Selektivität von 56,1 % zu Methylmethacrylat und 40,6 % zu Propylen.

Aus der US-A-4 249 019 ist ein Katalysator zur Herstellung von Carbonsäureestern durch Umsetzung von Aldehyden mit Alkanolen in Gegenwart von Sauerstoff bei 0 bis 200°C bekannt, der a) Palladium, b) ein Oxid, Hydroxid, Carbonat, Nitrat oder Carbonsäuresalz von Blei, Thallium oder Quecksilber und c) ein Oxid, Hydroxid, Carbonat oder Carbonsäuresalz eines Alkali- oder Erdalkalimetalls enthält. Mit einem solchen Katalysator erhält man zwar hohe Methylmethacrylat-Selektivitäten (90 bis 95 %), jedoch läßt die Raum-Zeit-Ausbeute, ausgedrückt als Produktivität (g MMA pro g Pd pro h), zu wünschen übrig. Dazu muß beachtet werden, daß die Zahlenangaben für die Produktivität in der Tabelle 1 bei niedrigem Umsatz ermittelt wurden (s. letzte Zeile der Anmerkung zur Tabelle) und daher kein reales Bild der Produktivität in bezug auf den Gesamtumsatz geben; bezogen auf den Gesamtumsatz zu Methylmethacrylat liegt die Produktivität zwischen 2 und 10,5.

Gegenstand der gemäß Art. 54(3) zu herücksichtigenden, am 28.09.1983 veröffentlichten Patentanmeldung EP-A-89 587 ist ein Katalysator, der Palladium und Blei als aktive Bestandteile enthält, wobei die aktiven Bestandteile sich auf einem Träger befinden, der wenigstens zwei der Oxide ZnO, Al$_2$O$_3$, La$_2$O$_3$ und TiO$_2$ enthält, wobei der Katalysator praktisch frei von Alkali- und Erdalkaliverbindungen ist. Das Palladium wird dabei vorzugsweise in Form einer salzsauren Palladiumchlorid-Lösung auf den Träger gebracht und dort zu metallischem Palladium reduziert. Hierdurch können die obenerwähnten Mängel vermieden werden. Anstelle von Palladiumchlorid können auch andere Salze des Palladiums, insbesondere wasserlösliche Salze z. B. das Nitrat, Acetat, Sulfat und ferner Komplexsalze wie Palladiumtetrammoniumchlorid. Es wurde nun gefunden, daß Katalysatoren für die Herstellung von Carbonsäureestern aus Aldehyden und Alkoholen in Gegenwart von Sauerstoff, die als aktive Bestandteile Palladium und Blei enthalten, wobei die aktiven Bestandteile sich auf einem Träger befinden, aktiv und selektiv sind, wenn der Träger allein aus Zinkoxid besteht und das Palladium als Palladiumsalz aus salzsaurer Lösung außgebracht und dann durch Reduktion freigesetzt ist. Mit den neuen Katalysatoren können z. B. bei Umsätzen von etwa 75 % und Selektivitäten von etwa 86 % Produktivitäten von 6,5 bis 7 g Methylmethacrylat je g Palladium und Stunde erzielt werden; bei einem Umsatz von 65 % und einer Selektivität von 91 % steigt die Produktivität auf etwa 10 g Methylmethacrylat / g Pd. h. Wenn beim Einsatz des neuen Katalysators bei einem Umsatz von weniger als 70, z. B. von 65 % oder weniger, gearbeitet wird, so wird eine 2-stufige Reaktionsführung bevorzugt, bei der z. B. zwei Reaktoren derart hintereinander geschaltet werden, daß der Austrag des ersten Reaktors nach Zugabe von weiterem Methacrolein dem zweiten Reaktor zugeführt wird, in dem dann das Gemisch weiter oxidiert wird.

Als Träger für die neuen Katalysatoren kann handelsübliches Zinkoxid, z. B. in Form von handelsüblichen Zylinderchen einer Lange von 2 bis 8 mm und eines Durchmessers von 2 bis 4 mm eingesetzt werden. Außerdem kann handelsübliches Zn(OH)$_2$ZnCO$_3$ eingesetzt werden, das nach Calcinieren zwischen 200 und 800°C, inbesondere 300 bis 500°C zu Zylinderchen z. B. einer Läge von 2 bis 8 mm und eines Durchmessers von 2 bis 4 mm verformt wird. Für die Herstellung der Katalysatoren sind geformte Teilchen aus Zinkoxid von besonderem Interesse, deren innere Oberfläche von 10 bis 100 m² je g, bestimmt nach BET, beträgt. Das Verfahren zur Bestimmung der BET-Oberfläche ist erläutert in R. Haul und G. Dümbgen, Chem.-Ing.-Technik 35, 586-589 (1963). Das vorgeformte ZnO-Trägermaterial, z. B. die Zylinderchen enthalten das Palladium in metallischer Form in einer Menge von meist 0,1 bis 10 Gew.-%, vorzugsweise von 0,2 bis 5 Gew.-% und besonders bevorzugt von 0,3 bis 2 Gew.-%, bezogen auf das Gesamtgewicht einschließlich Träger. Auch das Blei kann in dem Katalysator in metallischer Form oder aber in Form von Bleiverbindungen in einer Menge von 0,1 bis 20 Gew.-%, vorzugsweise von 0,2 bis 10 Gew.-% und insbesondere von 0,2 bis 4 Gew.-%, berechnet als Metall und bezogen auf das Gesamtgewicht, vorhanden sein. Man kann den Katalysator in an sich bekannter Weise herstellen, beispielsweise indem man den Träger zunächst mit einer wäßrigen Lösung von Palladiumchlorid unter Zusatz freier Salzsäure und dann mit einer wäßrigen Lösung eines Bleisalzes, wie Bleiacetat, solange behandelt, bis die Salze von dem Träger aufgenommen sind und zwischen oder nach den beiden Imprägnierungen eine Behandlung mit einem Reduktionsmittel, wie Wasserstoff oder Formaldehyd, vornehmen und den fertigen Katalysator schließlich trocknen.

Beim Behandeln der ZnO-Trägermaterialien mit einer wäßrigen salzsauren Palladiumsalz-Lösung soll diese Lösung im allgemeinen 0,05 bis 10 Gew.-%, insbesondere 0,1 bis 8 Gew.-% bezogen auf die Lösung, freie Salzsäure enthalten. Verwendet man eine Palladiumsalz-Lösung mit einem geringeren Gehalt an freier Salzsäure, so hat es sich als zweckmäßig erwiesen, die Trägermaterialien vor dem Imprägnieren mit der Palladiumsalz-Lösung oder gegebenenfalls auch die Katalysatorteilchen nach der Reduktion des Palladiumsalzes mit verdünnter Salzsäure zu behandeln, die vorzugsweise eine Konzentration von 0,05 bis 10, insbesondere von 0,1 bis 8 Gew.-%, haben soll.

Der neue Katalysator eignet sich mit besonderem Vorteil zur Herstellung von Carbonsäureestern, insbesondere α, β-olefinisch ungesättigter Carbonsäureestern, aus den entsprechenden Aldehyden und Alkanolen in Gegenwart von Sauerstoff. Die Alkanole können dabei im allgemeinen 1 bis 4 C-Atome enthalten und geradkettig oder verzweigt sein. Mit besonderem Vorteil kann er für die Herstellung von Metylmethacrylat aus Methacrolein, Methanol und Sauerstoff bei Temperaturen von im allgemeinen 0 bis 100, insbesondere von 30 bis 60° C, verwendet werden. Dabei kann in der Gasphase oder vorzugsweise in flüssiger Phase gearbeitet werden.

Die erfindungsgemäßen Katalysatoren können in diskontinuierlichem oder kontinuierlichem Betrieb eingesetzt werden. Dabei haben sich allgemein hierfür übliche Molverhältnisse von Alkanol, insbesondere Methanol, zu Aldehyd, insbesondere Methacrolein, von 200 : 1 bis 1 : 1, bewährt. Im allgemeinen arbeitet man dabei mit einer Katalysatormenge, die im Bereich des 0,2 bis 10-fachen des Gewichts des umzusetzenden Aldehyds gewählt wird, wenn man diskontinierlich arbeitet. Bei kontinuierlicher Fahrweise kann die Katalysatormenge bis zum 100-fachen des Gewichts des je Stunde durch den Reaktionsraum geleiteten Aldehyds betragen, mindestens jedoch soll die Menge etwa gleich groß sein.

Arbeitet man bei der Umsetzung des Aldehyds mit dem Alkanol, was vorgezogen wird in flüssiger Phase, so können Lösungsmittel, die gegen die Reaktionspartner indiffert sind, zugesetzt werden. Als solche kommen z. B. Octan, Toluol, Xylole und Schwerbenzin (Siedebereich 150-180° C) in Frage. Die Menge an derartigen zusätzlichen Lösungsmitteln soll jedoch im allgemeinen nicht größer sein als das 0,5-fache der eingesetzten Alkanol-Menge.

Als Sauerstoff setzt man molekularen Sauerstoff in reiner Form oder in Form eines Gemisches mit einem oder mehreren anderen Gasen, wie besonders Stickstoff oder auch Kohlendioxid, ein. So ist beispielsweise Luft als Sauerstoffquelle geeignet. Es hat sich bewährt, den Sauerstoff in größerer als für die Reaktion erforderlicher Menge einzusetzen. Vorzugsweise wird er in wenigstens der 1,5-fach stöchiometrischen Menge angewandt.

Die Umsetzung kann unter vermindertem, normalem oder erhöhtem Druck durchgeführt werden, wobei im allgemeinen Normaldruck bevorzugt wird.

Die in den folgenden Beispielen angegebenen Teile und Prozente beziehen sich auf das Gewicht. Es bedeuten darin weiter:

$$\text{Umsatz (\%)} = \frac{\text{umgesetztes Methacolein in Mol}}{\text{zugeführtes Methacrolein in Mol}} \times 100$$

$$\text{Selektivität (\%)} = \frac{\text{gebildetes Methylmethacrylat in Mol}}{\text{umgesetztes Methacrolein in Mol}} \times 100$$

**Beispiel 1**

100 Teile handelsübliche ZnO-Zylinderchen mit der spez. Oberfläche 20 bis 30 m²/g und einer Länge von 4 bis 6 mm und eines Durchmessers von 4 mm werden mit einer Lösung von 0,84 Teilen Palladiumchlorid in 500 Teilen Wasser (die durch Hydrolyse freie Salzsäure enthält) unter gelegentlichem Umrühren solange behandelt, bis die überstehende Lösung farblos klar ist. Man dekantiert dann das Wasser ab und behandelt die mit Palladiumchlorid imprägnierten ZnO-Trägerteilchen mit 500 Teilen einer 0,5 %-igen wäßrigen Formaldehyd-Lösung 4 Stunden bei 60° C. Man dekantiert dann ab und behandelt die Katalysatorteilchen 4 Stunden mit einer Lösung von 0,5 Teilen Bleiacetat in 500 Teilen Wasser, dekantiert wieder das Wasser ab und trocknet den Katalysator 12 Stunden unter Stickstoff bei 120° C. Aus der Analyse ergibt sich, daß der erhaltene Katalysator 0,21 Gew.-% Palladium und 0,32 Gew.-% Blei enthält.

100 Teile des Katalysators füllt man in ein mit Thermostat versehenes Reaktionsrohr einer Länge von 0,8 m und eines Durchmesser von 1,4 cm. Man hält die Temperatur des Reaktionsrohres bei 40° C und leitet von seinem unteren Ende aus stündlich 3 Teile Methacrolein, 27 Teile Methanol und 3 Teile Sauerstoff ein. Nach 24 Std. Betrieb beträgt der Umsatz 36 %, die Selektivität 85 % und die Produktivität 6,0 Teile Methylmethacrylat/Teil Pd. h. Nach einer Betriebsdauer von 120 Std. beträgt der Umsatz 42 %, die Selektivität 84 % und die Produktivität 7,2 Teile Methylmethacrylat/ Teil Pd. h.

**Beispiel 2**

Man arbeitet wie in Beispiel 1 angegeben, setzt aber zu der Palladiumchlorid-Lösung 1,2 Teile HCl zu. Man erhält dann unter sonst gleichen Bedingungen einen Katalysator, der 0,42 Gew.-% Palladium und 0,38 Gew.-% Blei enthält und der beim Umsatz von Methacrolein mit Methanol und Sauerstoff und unter den in Beispiel 1 genannten Bedingungen nach 24 Std. einen Umsatz von 78 %, eine Selektivität von 83 % und eine Produktivität von 6,6 Teilen Methylmethacrylat je Teil Pd . h, was nach 200 Std. Betrieb einen Umsatz von 74 %, eine Selektivität von 86 % und eine Produktivität von 6,5 Teilen Methylmethacrylat/Teil Pd . h ergibt.

**Beispiel 3**

Man arbeitet wie in Beispiel 1 angegeben, setzt aber eine Palladiumchlorid-Lösung ein, die 1,9 Teile Salzsäure enthält. Man erhält dann unter sonst gleichen Bedingungen einen Katalysator, der 0,45 Gew.-% Palladium und 0,31 Gew.-% Blei enthält.

50 Teile dieses Katalysators füllt man in ein mit Thermostat versehenes Reaktionsrohr einer Länge von 0,4 m und eines Durchmessers von 1,4 cm. Vom unteren Ende des bei 45° C gehaltenen Reaktionsrohres werden je Stunde 3 Teile Methacrolein, 27 Teile Methanol und 3,0 Teile Sauerstoff zugefügt. Nach 24 Std. beträgt der Umsatz 62 %, die Selektivität 84 % und die Produktivität 8,9 Teile Methylmethacrylat/Teil Pd . h, nach 120 Std. Betrieb beträgt der Umsatz 60 %, die Selektivität 87 % und die Produktivität 9,2 Teile Methylmethacrylat/Teil Pd . h.

**Beispiel 4**

200 Teile handelsübliche ZnO-Zylinderchen, einer spez. Oberfläche von 20 bis 30 m$^2$/g und einer Länge von 4 bis 6 mm und eines Durchmessers von 4 mm werden mit einer Lösung von 1,68 Teilen Palladiumchlorid und 3,8 Teilen HCl in 1000 Teilen Wasser unter Umrühren behandelt, bis die überstehende Lösung farblos klar ist. Man dekantiert das Wasser ab und behandelt den zurückbleibenden Katalysator 4 Std. bei 60° C mit 1000 Teilen einer 0,5 %-igen wäßrigen Formaldehyd-Lösung. Nach dem Abtrennen der wäßrigen Phase wird der Katalysator mit einer Lösung von 1 Teil Bleiacetat in 1000 Teilen Wasser 4 Stunden bei Raumtemperatur behandelt. Man dekantiert die überstehende Lösung ab und trocknet den zurückbleibenden Katalysator 12 Stunden unter Stickstoff bei 120° C. Erhalten wird ein Katalysator, der 0,42 % Palladium und 0,32 % Blei enthält.

65 Teile dieses Katalysators füllt man in ein mit Thermostat versehenes Reaktionsrohr (Reaktor 1) von 0,6 m Länge und 1,4 cm Durchmesser. Dieser Reaktor 1 wird durch einen Überlauf mit dem unteren Ende eines Reaktors 2 verbunden, der ebenso wie Reaktor 1 ausgestattet ist und 65 Teile des Katalysators enthält. Man leitet nun vom unteren Ende des Reaktors 1 stündlich bei 40° C 3 Teile Methacrolein, 57 Teile Methanol und 3 Teile Sauerstoff zu. Dem Austrag von Reaktor 1 werden 3 Teile Methacrolein je Stunde zugesetzt und diese Mischung unter Zugabe von 2 Teilen Sauerstoff je Stunde kontinuierlich dem unteren Ende von Reaktor 2 zugeführt der eine Temperatur von 40° C hat. Nach 24 Std. beträgt der Umsatz 68 %, die Selektivität 89 %, die Produktivität 8,9 g Methylmethacrylat je Teil Pd . h. Nach 170 Std. beträgt der Umsatz 65 %, die Selektivität 91 % und die Produktivität 9,3 g Methylmethacrylat/ Teil Pd . h.

**Beispiel 5**

In ein mit Thermostat versehenes Reaktionsrohr von 0,6 m Länge und 1,4 cm Durchmesser werden 65 Teile des in Beispiel 4 beschriebenen Katalysators eingefüllt und vom unteren Ende des bei 40° C gehaltenen Reaktionsrohres stündlich 3 Teile Methacrolein, 27 Teile Methanol und 3 Teile Sauerstoff zugeführt. Nach 24 Std. beträgt der Umsatz 65 %, die Selektivität 88 % und die Produktivität 8,3 Teile Methylmethacrylat/Teil Pd . h; nach 170 Std. Betrieb beträgt der Umsatz 66 %, die Selektivität 79 % und die Produktivität 8,3 g Methylmethacrylat/je Teil Pd . h.

**Beispiel 6**

500 Teile handelsübliches Zn(OH)$_2$ . ZnCO$_3$ werden 12 Std. bei 500° C calciniert, mit 200 Teilen Wasser 1 Std. im Kneter vermischt und zu Strängen von 3 mm Durchmesser und 4 bis 8 mm Länge verformt. Diese werden anschließend 2 Std. bei 200° C, 2 Std. bei 400° C und 2 Std. bei 500°C getrocknet. Man arbeitet wie in Beispiel 3 angegeben und erhält dann unter sonst gleichen Bedingungen einen Katalysator, der 0,49 Gew.-% Palladium und 0,40 Gew.-% Blei enthält.

50 Teile dieses Katalysators füllt man in ein mit Thermostat versehenes Reaktionsrohr von 0,5 m Länge (Durchmesser 1,4 cm) vom unteren Ende des bei 40°C gehaltenen Reaktionsrohres werden je Stunde 3 Teile Methacrolein, 27 Teile Methanol und 3 Teile Sauerstoff zugefügt. Nach 24 Std. beträgt der Umsatz 59 %, die Selektivität 90 % und die Produktivität 9,1 Teile Methylmethacrylat/Teil Pd . h, nach 170 Std. Betrieb beträgt der Umsatz 60 %, die Selektivität 91 % und die Produktivität 9,1 Teile Methylmethacrylat/Teil Pd . h.

**Patentansprüche**

1. Katalysator für die Herstellung von Carbonsäureestern aus Aldehyden und Alkanolen in Gegenwart von Sauerstoff, der als aktive Bestandteile Palladium und Blei enthält, wobei die aktiven Bestandteile sich auf einem Träger befinden, dadurch gekennzeichnet, daß der Träger allein aus Zinkoxid besteht und das Palladium als Palladiumsalz in salzsaurer Lösung aufgebracht und dann durch Reduktion freigesetzt ist.

2. Verwendung eines Katalysators nach Anspruch 1 zur Herstellung von Methylmethacrylat aus Methacrolein, Methanol und Sauerstoff bei Temperaturen von 0 bis 100°C.

3. Verwendung gemäß Anspruch 2 in flüssiger Phase.

**Claims**

1. A catalyst for the preparation of a carboxylate from an aldehyde and an alkanol in the presence of oxygen, which contains palladium and lead as active components, these being present on a carrier, wherein the carrier is composed solely of zinc oxide, and the palladium is applied as a palladium salt in a solution containing hydrochloric acid and is then liberated by reduction.

2. Use of a catalyst as claimed in claim 1 for preparing methyl methacrylate from methacrolein, methanol and oxygen at from 0 to 100°C.

3. Use as claimed in claim 2, in the liquid phase.

**Revendications**

1. Catalyseur pour la préparation de carboxylates à partir d'aldéhydes et d'alcanols en présence d'oxygène, contenant comme ingrédients actifs du palladium et du plomb déposés sur un support, caractérisé en ce que le support proprement dit est en oxyde de zinc, sur lequel le palladium est déposé sous forme d'un sel de palladium en solution chlorhydrique, le palladium libre étant ensuite obtenu par une réduction.

2. Utilisation du catalyseur suivant la revendication 1 pour la préparation de méthacrylate de méthyle à partir de méthacroléine, d'alcool méthylique et d'oxygène à des températures comprises entre 0 et 100°C.

3. Utilisation suivant la revendication 2 en phase liquide.